# EUROPEAN PATENT APPLICATION

(11) **EP 4 257 233 A1**
(43) Date of publication of application: **11.10.2023**
(21) Application number: 21900676.4
(22) Date of filing: 02.12.2021
(51) Int. Cl.: B01J 20/22, A61L 9/01, C07C 265/12

(54) **ACTIVE HYDROGEN-CONTAINING ORGANIC COMPOUND SCAVENGER, COMPOSITION, AND APPLICATIONS THEREOF**

(30) Priority: 02.12.2020 JP 2020200086; 30.07.2021 JP 2021125610
(71) Applicant: Tosoh Corporation, Yamaguchi 746-8501 (JP)
(72) Inventor: MIYAZAKI, Takanori, Shunan-shi Yamaguchi 746-8501 (JP); ARITA, Tomohiro, Shunan-shi Yamaguchi 746-8501 (JP); OKADA, Takeshi, Shunan-shi Yamaguchi 746-8501 (JP); KANBARA, Takeshi, Shunan-shi Yamaguchi 746-8501 (JP)
(74) Representative: TBK
(86) International application number: PCT/JP2021/044313
(87) International publication number: WO 2022/118926

(57) **Abstract**

The present invention provides a material for efficiently removing active hydrogen-containing compounds such as alcohol compounds. The present invention uses an active hydrogen-containing organic compound scavenger having a bromine content of from 38 to 78 wt% represented by the formula (1) (wherein R¹ and R² each independently represent a hydrogen atom or a C₁-₄ alkyl group, each R³ independently represents a hydrogen atom, a C₁₋₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).

## Description

### TECHNICAL FIELD

The present invention relates to a scavenger and a composition for removing active hydrogen-containing organic compounds and application thereof.

### BACKGROUND ART

Volatile organic compound (hereinafter abbreviated as VOC) scavengers and techniques for removing VOCs are used in various scenes of daily life. For example, the encapsulation ability of cyclodextrins and neutralization with amino acids are utilized in conventional techniques to remove VOCs responsible for sick building syndrome, such as amine compounds and aromatic hydrocarbon compounds. These techniques clean indoor air of airborne VOCs by way of spraying scavengers into the atmosphere. Other scavengers such as deodorant filters comprising activated carbon are also developed utilizing the ability of porous inorganic materials to adsorb various VOCs.

Meanwhile, with growing safety awareness against chemical substances, alcoholic VOCs are spotlighted in recent years. For example, in Japan, addition of two alcoholic chemical substances (2-ethyl-1-hexanol and texanol) to the list of VOCs which cause sick building syndrome is discussed. These alcohol compounds are known to be emitted from deteriorating resins in building materials and components in water-based paints, and no techniques have been established for removal of these compounds. Alcoholic VOCs which can be produced by microorganisms in the course of proliferation or metabolism such as 2-methyl-1-butanol and 1-pentanol are responsible for so-called moldy odors (Non-Patent Document 1).

### PRIOR ART DOCUMENTS

### NON-PATENT DOCUMENTS

Non-Patent Document 1: Journal of the Society of Odor and Fragrance Environment, 184-190, vol. 43, 2012

### DISCLOSURE OF INVENTION

### TECHNICAL PROBLEM

The present invention aims to provide a scavenger for active hydrogen-containing organic compounds which are difficult to remove by conventional techniques such as alcohol compounds.

### SOLUTION TO PROBLEM

As a result of their extensive research to attain the above-identified object, the present inventors found that the undermentioned scavenger or the undermentioned composition can remove active hydrogen-containing organic compounds which are difficult to remove by conventional techniques such as alcohol compounds and accomplished the present invention.

Namely, the present invention provides the following [1] to [18].

[1] An active hydrogen-containing organic compound scavenger comprising an isocyanate compound having a bromine content of from 38 to 78 wt% represented by the following formula (1): 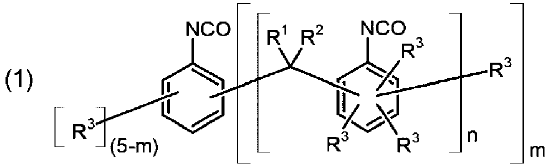 (wherein R¹ and R² each independently represent a hydrogen atom or a C₁₋₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).
[2] The active hydrogen-containing organic compound scavenger according to [1], wherein the isocyanate compound represented by the formula (1) (which may be a single species or a mixture of two or more species, wherein in the case of a mixture, n represents an average of the mixture) is an isocyanate compound represented by the following formula (1 a): 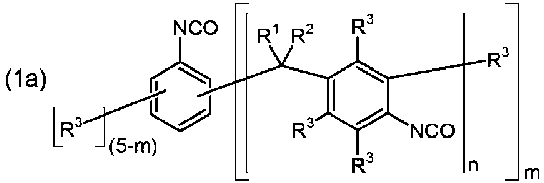 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).
[3] The active hydrogen-containing organic compound scavenger according to [1], wherein the isocyanate compound represented by the formula (1) (which may be a single species or a mixture of two or more species, wherein in the case of a mixture, n represents an average n of the mixture) is an isocyanate compound represented by the following formula (1b): 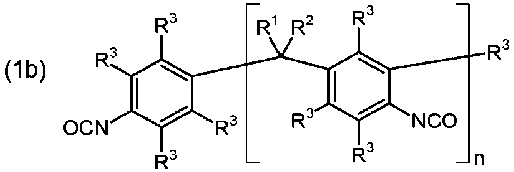 (wherein R¹ and R² each independently represent a hydrogen atom or a C₁-₄ alkyl group, each R³ independently represents a hydrogen atom, a C₁-₄ alkyl group or a bromine atom, and n represents a real number of 0 or more) (wherein the isocyanate compound may be a single species or a mixture of two or more species, and in the case of a mixture, n represents an average n of the mixture).
[4] The active hydrogen-containing organic compound scavenger according to any one of [1] to [3], wherein the bromine content is 50 to 78 wt%.
[5] The active hydrogen-containing organic compound scavenger according to any one of [1] to [4], wherein R¹ and R² each independently represent a hydrogen atom or a methyl group.
[6] The active hydrogen-containing organic compound scavenger according to any one of [1] to [5], wherein R¹ and R² each represent a hydrogen atom.
[7] The active hydrogen-containing organic compound scavenger according to any one of [1] to [6], wherein each R³ in the formula (1) independently represents a hydrogen atom or a bromine atom.
[8] The active hydrogen-containing organic compound scavenger according to any one of [1] to [7], wherein n represents a real number of from 0 to 3.
[9] A method for removing at least one compound selected from the group consisting of alcohol compounds, thiol compounds, amine compounds, phenol compounds and carboxylic acid compounds, which comprises bringing the at least one compound into contact with the active hydrogen-containing organic compound scavenger as defined in any one of [1] to [8].
[10] A composition comprising an isocyanate compound having a bromine content of from 38 to 78 wt% represented by the following formula (1): 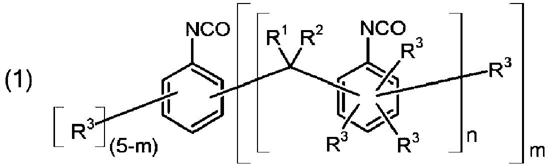 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture) and a support.
[11] The composition according to [10], wherein the isocyanate compound represented by the formula (1) (which may be a single species or a mixture of two or more species, and in the case of a mixture, wherein n represents an average n of the mixture) is an isocyanate compound represented by the following formula (1a): 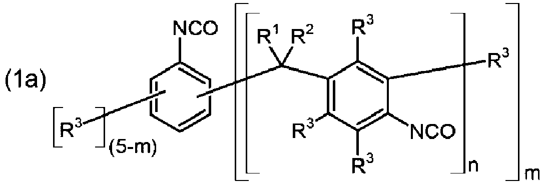 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).
[12] The composition according to [10], wherein the isocyanate compound represented by the formula (1) (which may be a single species or a mixture of two or more species, wherein in the case of a mixture, n represents an average n of the mixture) is an isocyanate compound represented by the following formula (1b): 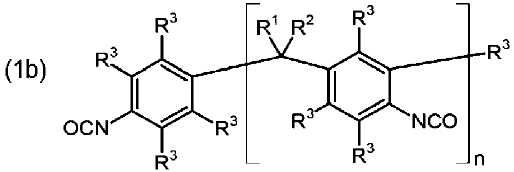 (wherein R¹ and R² each independently represent a hydrogen atom or a C₁-₄ alkyl group, each R³ independently represents a hydrogen atom, a C₁-₄ alkyl group or a bromine atom, and n represents a real number of 0 or more) (wherein the isocyanate compound may be a single species or a mixture of two or more species, and in the case of a mixture, n represents an average n of the mixture).
[13] The composition according to any one of [10] to [12], wherein each R³ independently represents a hydrogen atom or a bromine atom.
[14] The composition according to any one of [10] to [13], wherein the isocyanate compound is supported on the support.
[15] The composition according to any one of [10] to [14], wherein the amount of the isocyanate compound supported on the support is from 0.1 part by weight to 60 parts by weight per 100 parts by weight of the support.
[16] The composition according to any one of [10] to [15], wherein the support comprises one or members selected from the group consisting of activated carbon, activated clay, diatomaceous earth, a porous resin, non-woven cloth, mesoporous silica, silica gel, aluminosilicate, hydrotalcite, zeolite, activated alumina, titania, magnesia and zirconia.
[17] An active hydrogen-containing organic compound scavenger comprising the composition as defined in any one of [10] to [16].
[18] A method for removing at least one compound selected from the group consisting of alcohol compounds, thiol compounds, amine compounds, phenol compounds and carboxylic acid compounds, which comprises bringing the at least one compound into contact with the composition as defined in any one of [10] to [16].

### ADVANTAGEOUS EFFECTS OF INVENTION

The scavenger or composition of the present invention can remove active hydrogen-containing organic compounds such as alcohol compounds more selectively than the conventional techniques.

The scavenger or composition of the present invention binds to active hydrogen-containing organic compounds through chemical reactions with isocyanate groups and hence does not recontaminate the air by releasing the captured active hydrogen-containing organic compounds. Because of its high water resistance, its ability to capture active hydrogen-containing organic compounds lasts long in living environments.

### DESCRIPTION OF EMBODIMENTS

Now, the present invention will be described in detail.

The present invention relates to the above-mentioned scavenger or composition and their applications. Herein, wt% denotes percent by weight, and from A to B denotes at least A and at most B (the same applies hereinafter).

In the formulae (1), (1a) and (1b), each R³ independently represents a hydrogen atom, a C₁₋₄ alkyl group or a bromine atom.

The C₁₋₄ alkyl group as R³ is not particularly limited and may, for example, be a methyl group, an ethyl group, a propyl group, an isopropyl group, a cyclopropyl group, a butyl group, an isobutyl group, a sec-butyl group or a tert-butyl group.

Each R³ preferably independently represents a hydrogen atom, a methyl group or a bromine atom, more preferably independently a hydrogen atom or a bromine atom, in view of efficient production of the isocyanate compound represented by the formula (1), (1a) or (1b).

When each R³ is independently a hydrogen atom or a bromine atom, the formula (1) corresponds to the following formula (1'). 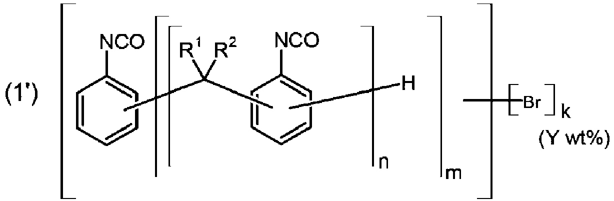 (wherein R¹ and R² each independently represent a hydrogen atom or a C₁₋₄ alkyl group, m represents at least one selected from the group consisting of 0, 1, 2 and 3, n represents a real number of 0 or more, k represents a real number from 1 to [5-m+m×(3×n+1)] (namely, from 1 to [5+3×m×n]), and Y wt% represents the bromine content of the isocyanate compound in a condition that the real number (k) of the bromine atoms are attached to the aromatic ring carbon atoms.)

The formula (1') means by -Br, which does not necessarily represent one bromine atom, that the bromine atoms attached to aromatic ring(s) corresponds to Y wt%, which is defined below. Y wt% is the bromine content of the isocyanate compound (which may be a single species or a mixture of two or more species) and is preferably from 38 to 78 wt%, as mentioned previously.

The isocyanate compound (which may be a single species or a mixture of two or more species, and in the case of a mixture, n represents an average n of the mixture) is preferably an isocyanate compound represented by the formula (1a) (which may be a single species or a mixture of two or more species, and in the case of a mixture, n represents an average n of the mixture), more preferably an isocyanate compound represented by the formula (1b) (which may be a single species or a mixture of two or more species, and in the case of a mixture, n represents an average n of the mixture), in view of its ability to capture active hydrogen-containing organic compounds.

In the formulae (1), (1'), (1a) and (1b), R¹ and R² each independently represent a hydrogen atom or a C₁-₄ alkyl group.

The C₁₋₄ alkyl group as R¹ or R² is not particularly limited and may, for example, be a methyl group, an ethyl group, a propyl group, an isopropyl group, a cyclopropyl group, a butyl group, an isobutyl group, a sec-butyl group or a tert-butyl group.

It is preferred that R¹ and R² each independently represent a hydrogen atom or a methyl group, and is more preferred that R¹ and R² each represent a hydrogen atom, in view of efficient production of the isocyanate compound.

In the formulae (1), (1'), (1a) and (1b), n represents a real number of 0 or more. n is preferably a real number larger than 0, more preferably a real number larger of 0.1 or more, more preferably a real number larger than 0.3, in view of efficient production of the isocyanate compound represented by the formulae (1), (1'), (1a) or (1b). n is preferably a real number of at most 10, more preferably a real number of at most 6, more preferably a real number of at most 3, more preferably a real number of at most 2, in view of efficient production of the isocyanate compound represented by the formula (1), (1'), (1a) or (1b).

n is preferably a real number of from 0 to 10, more preferably a real number of from 0 to 6, more preferably a real number larger than 0 and not larger than 6, more preferably a real number of from 0.1 to 3, more preferably a real number of larger than 0.3 and not larger than 2, in view of the ability of the scavenger or composition of the present invention to capture active hydrogen-containing organic compounds. It is more preferably a real number of from 0 to 3, more preferably a real number of from 0 to 2, in view of efficient production of the isocyanate compound represented by the formula (1), (1'), (1a) or (1b).

The formula (1), (1'), (1a) or (1b) means that the group written within []n does not exist when n is 0.

In the formulae (1), (1') and (1a), m represents at least one selected from 0, 1, 2 and 3. Namely, it follows that the isocyanate represented by the formulae (1), (1') or (1a) may be a single species wherein m=0, or may be a mixture of a species wherein m=0, a species wherein m=1, a species wherein m=2, and a species wherein m=3.

m is preferably at least one selected from the group consisting of 0, 1 and 2, more preferably at least one selected from the group consisting of 0 and 1, more preferably 0 or 1, in view of efficient production of the isocyanate compound represented by the formula (1), (1') or (1a).

The formula (1), (1') or (1a) means that the group written within []m does not exist when m is 0, and means that when m is 3, the number of the group written within []m is three each of which is bonded to the benzene ring at their bases.

When m is 2 or 3, there are two or three n's in the formula, and in this case, n is their average by definition.

For example, when m=0 (i.e., n=0), the isocyanate is called a mononuclear compound, and when m=1 and n=1, the isocyanate is called a binuclear compound, when m=1 and n=2, the isocyanate is called a trinuclear compound, when m=1 and n=3, or m=3 and n=1 (the average of three n's), the isocyanate is called a tetranuclear compound, and wherein m=1 and n=4, or m=3 and n=1.33 (=4/3 on average), the isocyanate is called a pentanuclear compound.

Some examples of mononuclear to pentanuclear compounds of the formula (1) and the like are shown below, although there are no particular restrictions thereto.

| | |
|---|---|
| Mononuclear compounds | 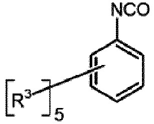 |
| Binuclear compounds | 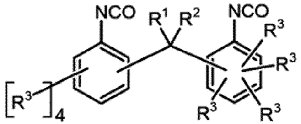 |
| Trinuclear compounds | 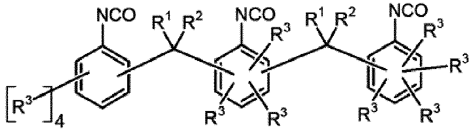 |
| Tetranuclear compounds | 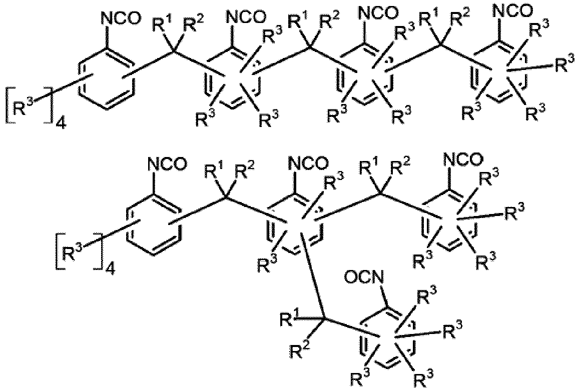 |
| Pentanuclear compounds | 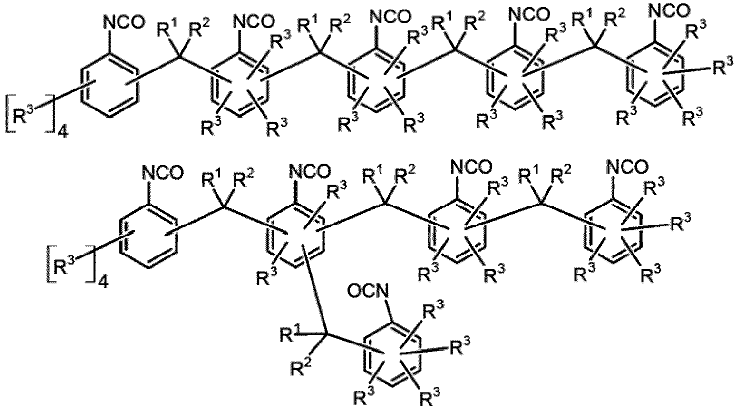 |

In the above formulae, the definitions of R¹, R² and R³ and their preferred range are the same as those previously mentioned.

The isocyanate compound of the present invention may be a single species or a mixture of species of isocyanate compound represented by the formula (1), (1'), (1a) or (1b) having different combinations of m and n. When the isocyanate compound represented by the formula (1), (1'), (1a) or (1b) is a mixture, n can be a real number other than integers because it is an average of n of each species in the mixture.

Namely, the isocyanate compound of the present invention may consist of mononuclear compounds only, binuclear compounds only or trinuclear compounds only, or may be a mixture of a mononuclear compound and a binuclear compound, a mixture of a mononuclear compound, a binuclear compound and a trinuclear compound, or a mixture of a mononuclear compound, a binuclear compound, a trinuclear compound and a tetranuclear compound, or a mixture of a tetranuclear or higher nuclear compound, without any restrictions.

When the isocyanate compound represented by the formula (1), (1'), (1a) or (1b) is a mixture of a plurality of compounds containing different numbers of nuclei, it is not necessary, though preferred, that each nuclear compound satisfies the bromine content (Y wt% in the formula (1')) prescribed above as long as the mixture satisfies the prescribed bromine content as a whole. Namely, for example, the compound of the present invention may be produced by mixing a binuclear compound having a bromine content below the prescribed range and a trinuclear compound having a bromine content above the prescribed range so that the resulting mixture has a bromine content within the prescribed range.

The isocyanate compound represented by the formula (1), (1'), (1a) or (1b) is preferably a mononuclear compound, a binuclear compound, a trinuclear compound, a tetranuclear compound or a mixture of compounds having different numbers of nuclei. When it is a mixture of compounds having different numbers of nuclei, it preferably comprises from 0 to 20% of a mononuclear compound, from 10 to 99% of a binuclear compound, from 5 to 60% of a trinuclear compound and from 0 to 30% of a tetranuclear or higher nuclear compound.

The ratio of each nuclear compound means the GPC area percentage, which is determined under the following conditions.

A GPC system HLC-8320GPC manufactured by Tosoh Corporation was equipped with columns manufactured by Tosoh Corporation, TSK gel guard column Super AW-H, TSK gel Super AW2500, TSK gel Super AW2500, TSK gel Super AW3000 and TSK gel Super AW3000, serially connected in this order, and was used at a column temperature of 40°C. As the eluent, tetrahydrofuran (THF) was flown at a flow rate of 0.6 mL/min, and as the detector, a UV-Vis absorptiometric detector was used at 254 nm. For data processing, GPC-8020 model II version 4.10, manufactured by Tosoh Corporation, was used, and 10 µL of a solution prepared by dissolving 0.1 g of a sample in 10 mL of THF was injected after filtration through a microfilter. From the resulting chromatogram, the content of each of mononuclear, binuclear, trinuclear, tetranuclear and higher nuclear compounds can be calculated.

When the isocyanate compound represented by the formula (1), (1'), (1a) or (1b) is a mixture of two or more species (having different numbers of nuclei), n is expressed as an average n (real number) of the mixture and is calculated from the ratio (GPC area percentage) of each nuclear compound.

For example, when the ratio of a binuclear compound (wherein m=1, and n=1) is 50 (GPC area percentage), and the ratio of a trinuclear compound (wherein m=1, and n=2) is 50 (GPC area percentage), the average n is calculated at (1×0.5+2×0.5)=1.5.

In the formula (1'), k represents a real number of from 1 to [5-m+m×(3×n+1)] (namely, a real number of from 1 to [5+3×m×n]). For example, when m=0 (i.e., n=0), k represents a real number of from 1 to 5, and when m=1 and n=1, k represents a real number of from 1 to 8, and when m=1 and n=2, k represents a real number of from 1 to 11.

k represents a real number because n is a real number, and because when the isocyanate compound represented by the formula (1), (1'), (1a) or (1b) is prepared, the number of bromine atoms added to each molecule is not necessarily the same. For example, when a compound wherein m=n=0 having one bromine atom and a compound wherein m=n=0 having two bromine atoms are present in a 50:50 ratio (molar ratio), k represents a real number of about 1.5.

The isocyanate compound represented by the formula (1), (1'), (1a) or (1b) is characterized by having a bromine content (Y wt% in the formula (1')) of from 38 to 78 wt%. The bromine content is preferably from 45 to 78 wt%, more preferably from 50 to 78 wt%, further preferably from 55 to 78 wt%, in view of selective removal of active hydrogen-containing organic compounds such as alcohol compounds. The bromine content of the isocyanate compound represented by the formula (1), (1'), (1a) or (1b) (Y wt% in the formula (1')) was measured by the oxygen flask combustion-IC method.

There is a correlation between the bromine content (Y wt% in the formula (1')) and the real number k, and the above-mentioned range of k is defined so as not to exceed the maximum number of bromine atoms the isocyanate compound represented by the formula (1').

The isocyanate compound represented by the formula (1), (1'), (1a) or (1b) can be produced by isocyanation of the corresponding amine compound, as in Patent Document (GB971168), which discloses production of bromine-containing aromatic isocyanate compounds.

The isocyanate compound represented by the formula (1), (1a) or (1b) (which may be a single species or a mixture of two or more species) can be produced, for example, by converting the amino group(s) in an amine compound represented by the formula (2), (2a) or (2b) (which may be a single species or a mixture of two or more species, and in the case of a mixture, n represents an average n of the mixture) to isocyanate group(s), although there are no particular restrictions. 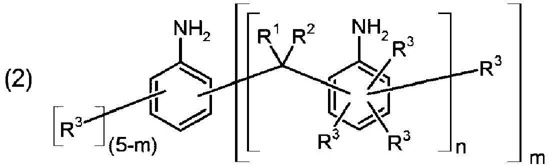 (wherein R¹ and R² each independently represent a hydrogen atom or a C₁-₄ alkyl group, each R³ independently represents a hydrogen atom, a C₁₋₄ alkyl group or a bromine atom, m represents at least one selected from the group consisting of 0, 1, 2 and 3, and n represents a real number of 0 or more) 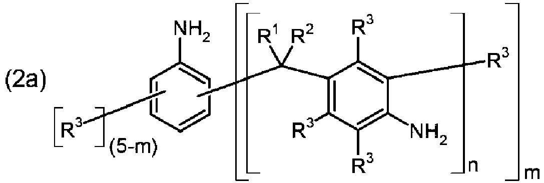 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) 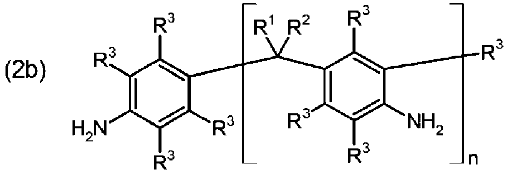 (wherein R¹ and R² each independently represent a hydrogen atom or a C₁-₄ alkyl group, each R³ independently represents a hydrogen atom, a C₁-₄ alkyl group or a bromine atom, and n represents a real number of 0 or more).

The definitions of R¹, R², R³, n and m in the formula (2), (2a) or (2b) and their preferred ranges are the same as those of R¹, R², R³, n and m in the formula (1) previously mentioned.

The bromine content (wt%) in the formula (2), (2a) or (2b) is hard to specify, but is preferred to be theoretically consistent with the bromine content (wt%) in the formula (1), (1a) or (2b).

The amine compound represented by the formula (2), (2a) or (2b) is not particularly limited and may, for example, be a bromination product of aniline, a bromination product of 4,4'-methylenedianiline, a bromination product of 2,2'-bis(4-aminophenyl)propane, a bromination product of a polymer of aniline and formalin or a bromination product of a polymer of aniline and acetone.

The amine compound represented by the formula (2), (2a) or (2b) may be a commercial product or may be produced by bromination of aniline, 4,4'-methylenedianiline, 2,2'-bis(4-aminophenyl)propane, a polymer of aniline and formalin or a polymer of aniline and acetone by well-known methods, or by polymerization of a bromoaniline and formalin or polymerization of a bromoaniline and acetone.

Next, the active hydrogen-containing organic compound scavenger or composition comprising an isocyanate compound represented by the formula (1), (1'), (1a) or (1b) will be described.

Isocyanate compounds (other than isocyanate compounds represented by the formula (1), (1'), (1a) or (1b)) such as diphenylmethane diisocyanate (MDI) and tolylene diisocyanate (TDI) are generally used as a raw material for production of polyurethans on the basis of the basic ability of the isocyanate group to react with nucleophilic functional groups (so-called active hydrogen groups such as hydroxy groups and amino groups). Isocyanate compounds in conventional use are so reactive as to react with atmospheric water even around room temperature (from 15°C to 40°C) and cannot be stored stably in the atmosphere.

Unlike these conventional isocyanates, the isocyanate compound represented by the formula (1), (1'), (1a) or (1b) has high water resistance and has the feature that it reacts with water very slowly around room temperature (from 15°C to 40°C) but reacts with active hydrogen-containing organic compounds such as alcohol compounds around room temperature (from 15°C to 40°C). Namely, in the presence of water and active hydrogen-containing organic compounds such as alcohol compounds, the isocyanate compound represented by the formula (1), (1'), (1a) or (1b) selectively reacts with active hydrogen-containing organic compounds such as alcohol compounds. This feature makes the active hydrogen-containing organic compound scavenger comprising an isocyanate compound represented by the formula (1), (1'), (1a) or (1b) or the composition comprising an isocyanate compound represented by the formula (1), (1'), (1a) or (1b) and a support, useful as a scavenger having long-lasting effect in living environments for removing active hydrogen-containing organic compounds such as alcohol compounds.

The target active hydrogen-containing organic compound to be removed is not particularly limited and may, for example, be an alcohol compound, a thiol compound, an amine compound, a phenol compound or a carboxylic acid compound, and specific examples include primary alcohols such as methanol, ethanol, 1-propanol, 2-methyl-1-propanol, 1-butanol, 2-methyl-1-butnol, 1-pentanol, 1-hexanol, 2-ethyl-1-hexanol, 1-hepanol, 1-octanol, 1-nonanol, 1-decanol, 1-undecanol, 1-dodecanol, 2-methoxyethanol and 2-ethoxyethanol, secondary alcohols such as 2-propanol, 2-butanol, 2-pentanol, 3-pentanol, 2-hexanol, cyclohexanol, 2,2,4-trimethylpentane-1,3-diol monoisobutyrate, tertiary alcohols such as tert-butanol, 2-methyl-2-pentanol, 3-methyl-3-pentanol, terpineol and linalool, polyols such as 1,2-ethylene glycol, 1,4-butanediol and glycerin, amines such as methylamine, dimethylamine, ethylamine, diethylamine, butylamine, hexylamine, octylamine, ethanolamine, diethanolamine, ethylenediamine, diethylenetriamine and hexamethylenediamine, thiol compounds such as methylmercaptan, ethylmercaptan, propylmercaptan, butylmercaptan, hexylmercaptan and thiophenol, carboxylic acids such as formic acid, acetic acid and propionic acid and phenol compounds such as phenol and salicylic acid.

Among these active hydrogen-containing organic compounds, alcohol compounds or carboxylic acid compounds are preferred because being able to remove these compounds which are difficult to remove by conventional techniques makes the effect of the present remarkable and distinctive.

The active hydrogen-containing organic compound as the target to be removed may be in a solid, liquid or gaseous state, preferably in a liquid or gaseous state, because efficient removal of active hydrogen-containing organic compounds requires frequent contact between the isocyanate compound represented by the formula (1), (1'), (1a) or (1b) and the target to be removed. The active hydrogen-containing organic compound as the target to be removed may be in the state of a solution in a solvent. The solvent is not particularly restricted and may be an aqueous solvent, a hydrocarbon solvent, a halogenated solvent, an aromatic solvent or an ether solvent.

The isocyanate compound represented by the formula (1), (1'), (1a) or (1b) is a liquid or a solid at ordinary temperature. The isocyanate compound represented by the formula (1), (1'), (1a) or (1b) itself may be used as a scavenger in a liquid or solid form, or the isocyanate compound represented by the formula (1), (1'), (1a) or (1b) in a solid state may be used in a powder form after crushed in a mortar into powder, or in the form of lumps after subsequent compression of the powder. The active hydrogen-containing organic compound scavenger of the present invention covers all these forms.

The composition comprising an isocyanate compound having a bromine content of from 38 to 78 wt% represented by the formula (1) and a support (hereinafter referred to as "the composition of the present invention") is also effective as the active hydrogen-containing organic compound, as intended by the present invention. In such a case, it is preferred that the isocyanate compound represented by the formula (1) is supported on the support.

The isocyanate compound having a bromine content of from 38 to 78 wt% represented by the formula (1) in the composition of the present invention is preferably an isocyanate compound having a bromine content (Y wt%) of from 38 to 78 wt% represented by the formula (1'), more preferably an isocyanate compound having a bromine content of from 38 to 78 wt% represented by the formula (1a), further preferably an isocyanate compound having a bromine content of from 38 to 78 wt% represented by the formula (1b).

The bromine content of the isocyanate compound represented by the formula (1), (1'), (1a) or (1b) is preferably from 45 to 78 wt%, preferably from 50 to 78 wt%, more preferably from 55 to 78 wt%, in view of selective removal of active hydrogen-containing organic compounds such as alcohol compounds. In the present invention, the bromine content (Y wt% in the formula (1')) is measured by the oxygen flask combustion-IC method.

The support may, for example, be activated carbon, activated clay, diatomaceous earth, a porous resin, non-woven cloth, mesoporous silica, silica gel, aluminosilicate, hydrotalcite, zeolite, activated alumina, titania, magnesia or zirconia, although there is no particular restriction. Among them, activated carbon, non-woven cloth, silica gel or hydrotalcite is preferred, silica gel or hydrotalcite is more preferred, to provide a high-performance scavenger.

The isocyanate compound represented by the formula (1), (1'), (1a) or (1b) may be loaded onto a support by mixing a solution of the isocyanate compound in an organic solvent with the support and removing the organic solvent. The organic solvent preferably has no active hydrogen and may, for example, be hexane, dichloromethane, chloroform, acetone, tetrahydrofuran, dioxane, benzene, chlorobenzene, toluene or xylene.

The composition of the present invention may further comprise a styrenebutadiene rubber, polyvinylidene fluoride, polytetrafluoroethylene, clay such as sepiolite or attapulgite or a binder such as sodium silicate or silica sol.

The amount of the isocyanate compound represented by the formula (1), (1'), (1a) or (1b) on the support is preferably from 0.1 part by weight to 60 parts by weight per 100 parts by weight of the support, more preferably from 0.5 part by weight to 40 parts by weight per 100 parts by weight of the support, more preferably from 1 part by weight to 25 parts by weight per 100 parts by weight of the support, for each of them.

As mentioned above, the isocyanate compound represented by the formula (1), (1'), (1a) or (1b) may be used as a scavenger in the form of powder or lumps or after mixed with a support or loaded onto a support. It is preferably supported on a support such as silica gel, zeolite, alumina, hydrotalcite or activated carbon when used as a scavenger, in view of more efficient removal of active hydrogen-containing organic compounds.

In the present invention, the operation temperature for removal of active hydrogen-containing organic compounds by using the active hydrogen-containing organic compound scavenger of the present invention or the composition of the present invention may be from 0 to 150°C without any particular restrictions, and it is preferably from 10 to 100°C, more preferably from 20 to 70°C for selective removal of active hydrogen-containing organic compounds such as alcohol compounds.

The active hydrogen-containing organic compound scavenger of the present invention or the composition of the present invention can be used for removal of active hydrogen-containing organic compounds, by bringing at least one compound selected from the group consisting of alcohol compounds, thiol compounds, amine compounds, phenol compounds and carboxylic acid compounds with the active hydrogen-containing organic compound scavenger of the present invention or the composition of the present invention.

The target compounds to be removed (active hydrogen-containing organic compounds) are preferably those generated in a natural or living environment, artificially or non-artificially.

Among these target compounds to be removed (active hydrogen-containing organic compounds), alcohol compounds or carboxylic acid compounds are preferred as being able to remove these compounds which are difficult to remove by conventional techniques makes the effect of the present invention remarkable and distinctive.

### BRIEF DESCRIPTION OF DRAWINGS

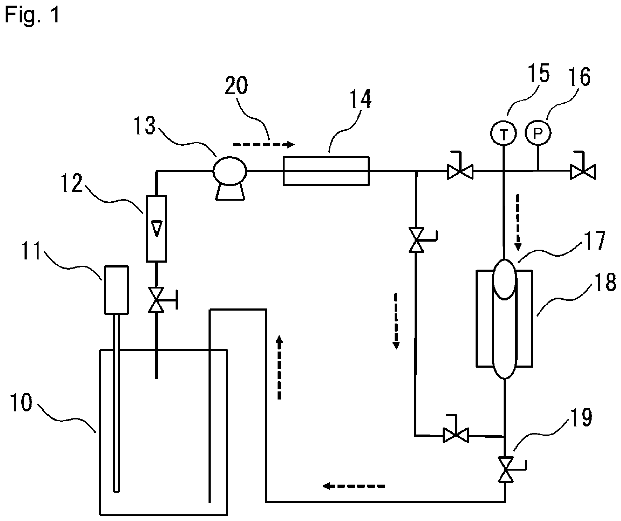

[Fig. 1] A schematic view of the system used to assess the removal of an active hydrogen-containing organic compound in Examples.

### EXAMPLES

Now, the present invention will be described in further detail with reference to Examples. However, it should be understood that the present invention is by no means restricted thereto.

### [GCMS analysis]

| | |
|---|---|
| Instrument: | Electronic nose system HERACLES II manufactured by Alpha M.O.S. Japan |
| Measurement conditions: | column = Agilent J&W GC column DB-5 |
| | vaporization chamber temperature = 220°C |
| | detector temperature = 260°C |
| | column temperature = 250°C |
| | heating rate = 1.5 °C/sec |

### [FT-IR analysis]

Instrument: Frontier MIR/NIR manufactured by PerkinElmer Inc.
Measurement conditions: ATR method, MIR mode

### [Determination of bromine content]

Combustion: the oxygen flask combustion method (in compliance with ISO7725-2020)
Instrument: IC-2001 manufactured by Tosoh Corporation

### SYNTHETIC EXAMPLE 1

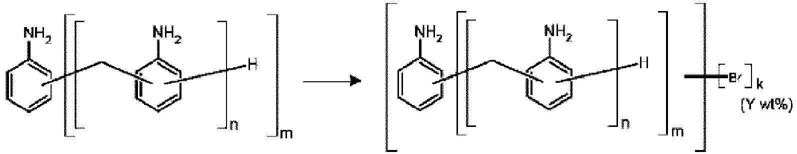

A 2 L separable flask was loaded with 100 g of a 4,4'-methylenedianiline composition obtained by condensation of aniline and formalin (comprising 65% of a binuclear product, 23% of a trinuclear product, 8% of a tetranuclear product and 4% of a pentanuclear or higher nuclear product), 5.00 g of ferric chloride and 700 mL of 1,2-dichloroethane. 338 g of bromine diluted with 300 mL of 1,2-dichloroethane was added dropwise to the 2 L separable flask over 1 hour with stirring, with an accompanying rise in the inner temperature to 50°C. The resulting solution was cooled to room temperature over 1 hour under stirring and then washed with aqueous hydrazine to remove the unreacted bromine until the pH came into the basic range. The 1,2-dichloroethane layer was separated from the aqueous layer and was added dropwise to methanol to precipitate a pale red solid. The solid was recovered by filtration and washed with methanol and dried to yield 153 g of a pale red solid (the bromination product of the 4,4'-methylenedianiline composition). The bromine content of the solid determined by the oxygen flask combustion-IC method was 59.6 wt%.

### SYNTHETIC EXAMPLE 2

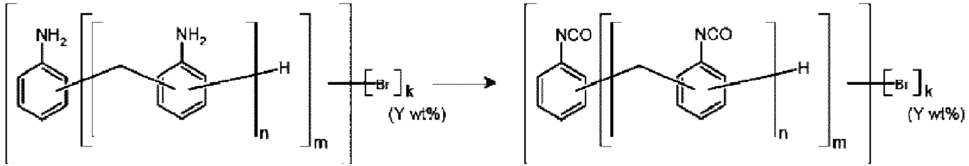

A 2 L separable flask equipped with a stirrer was loaded with 12.5 g of the pale red solid obtained in Synthetic Example 1 (the bromination product of the 4,4'-methylenedianiline composition) and 987.5 g of chlorobenzene under nitrogen and heated to 130°C, and 13.4 g of hydrogen chloride gas was blown into the resulting solution over 90 minutes to convert the brominating product to a salt. Then, 44 g of phosgene gas was blown into the solution over 2 hours, and after 2 hours of aging at 125°C, nitrogen gas was bubbled into the solution to remove the phosgene gas from the system until the absence of phosgene was confirmed. After cooling to room temperature, the reaction solution was filtered to remove the insoluble. The filtrate was concentrated on an evaporator, and the resulting concentrate was air-dried at 45°C to yield 12.4 g (yield 79%) of a mixture (hereinafter referred to as the product of Synthetic Example 2) of 4,4'-methylenebis(2,6-dibromoisocyanatobenzene) (binuclear compound) and its oligomers (comprising trinuclear, tetranuclear, pentanuclear and even higher nuclear compounds) as a brown powder. The NCO content (mass percentage of isocyanate groups to the total mass) was 14.5 mass%, and the bromine content was 54.3 wt%. The product of Synthetic Example 2 can be used as an active hydrogen-containing organic compound scavenger of the present invention, as described later.

### SYNTHETIC EXAMPLE 3

A 2 L separable flask equipped with a stirrer was loaded with 30.0 g of 4,4'-methylenebis(2-bromoanilie) (comprising 100% of a binuclear compound) and 1470.0 g of chlorobenzene under nitrogen and heated to 130°C. 12.3 g of hydrogen chloride gas was blown into the resulting solution over 120 minutes to convert 4,4'-methylenebis(2-bromoanilie) to a salt. Then, 109.6 g of phosgene gas was blown into the solution over 6 hours, and nitrogen gas was bubbled into the solution to remove the phosgene gas from the system until the absence of phosgene was confirmed. After cooling to room temperature, the reaction solution was filtered to remove the insoluble. The filtrate was concentrated on an evaporator, and the resulting concentrate was air-dried at 45°C to yield 33.0 g (yield 96%) of 4,4'-methylenebis(2-bromoisocyantobenzene) (hereinafter referred to as the product of Synthetic Example 3) as a white powder. The NCO content (mass percentage of isocyanate groups to the total mass) was 21.0 mass%, and the bromine content was 39.1 wt%. The product of Synthetic Example 3 can be used as an active hydrogen-containing organic compound scavenger of the present invention, as described later.

### SYNTHETIC EXAMPLE 4

A 300 mL three-necked flask was loaded with 5.00 g of a 4,4'-methylenedianiline composition (comprising 65% of a binuclear compound, 23% of a trinuclear compound, 8% of a tetranuclear compound and 4% of a pentanuclear or higher nuclear compound), 0.25 g of ferric chloride and 100 mL of methanol, and 19.5 g of bromine diluted with 25 mL of methanol was added dropwise to the 200 mL separable flask over 1 hour under stirring. The reaction solution was post-treated in the same manner as in Synthetic Example 1 to yield 12.0 g of a red-brown solid (the bromination product of the 4,4-methylenedianline composition) (hereinafter referred to as the product of Synthetic Example 4). The bromine content of the solid determined by the oxygen flask combustion-IC method was 58.5 wt%.

### SYNTHETIC EXAMPLE 5

A 300 mL three-necked flask was loaded with 10.0 g of 4,4'-methylenedianiline (comprising 100% of a binuclear compound), 0.50 g of ferric chloride and 200 mL of methanol, and 19.5 g of bromine diluted with 25 mL of methanol was added to the 200 mL separable flask over 1 hour under stirring. The reaction solution was post-treated in the same manner as in Synthetic Example 1 to yield 25.5 g of a beige solid (the bromination product of the 4,4-methylenedianline composition) (hereinafter referred to as the product of Synthetic Example 5). The bromine content of the solid determined by the oxygen flask combustion-IC method was 59.4 wt%.

### SYNTHETIC EXAMPLE 6

A 2 L separable flask equipped with a stirrer was loaded with 39.8 g of the product of Synthetic Example 5 (comprising 100% of a binuclear compound) and 1150.0 g of orthochlorobenzene under nitrogen and heated to 140°C, and 11.3 g of hydrogen chloride gas was blown into the resulting solution over 120 minutes to convert the product of Synthetic Example 5 to a salt. Then, 61.3 g of phosgene gas was blown into the solution over 4 hours, and nitrogen gas was bubbled into the solution to remove the phosgene gas from the system until the absence of phosgene was confirmed. After cooling to room temperature, the reaction solution was concentrated on an evaporator, and the resulting concentrate was air-dried at 45°C to yield 42.7 g of 4,4'-methylenebis(2,6-dibromoisocyanatobenzen) (hereinafter referred to as the product of Synthetic Example 6) as a brown powder. The NCO content (mass percentage of isocyanate groups to the total mass) was 14.3 mass%, and the bromine content was 56.0 wt%. The product of Synthetic Example 6 can be used as the active hydrogen-containing organic compound scavenger of the present invention, as described later.

### SYNTHETIC EXAMPLE 7

A 2 L separable flask equipped with a stirrer was loaded with 50.0 g of 4,4'-methylenebis(3-bromoaniline) (comprising 45% of a binuclear compound, 18% of a trinuclear compound, 17% of a tetranuclear compound and 20% of pentanuclear or higher nuclear compounds) and 1400.0 g of chlorobenzene under nitrogen and heated to 130°C. 20.0 g of hydrogen chloride gas was blown into the resulting solution over 120 minutes to convert 4,4'-methylenebis(3-bromoaniline) to a salt. Then, 110.0 g of phosgene gas was blown into the solution over 6 hours, and nitrogen gas was bubbled into the solution to remove the phosgene gas from the system until the absence of phosgene was confirmed. After cooling to room temperature, the reaction solution was concentrated on an evaporator, and the resulting concentrate was washed with small amounts of toluene and tetrahydrofuran. The resulting powder was air-dried to yield 40.7 g of 4,4'-methylenebis(3-bromoisocyanatobenzen) (hereinafter referred to as the product of Synthetic Example 7) as a brown powder. The bromine content was 38.3 wt%. The product of Synthetic Example 7 can be used as an active hydrogen-containing organic compound scavenger of the present invention, as described later.

The isocyanate compounds obtained above and the isocyanate compound (2,4,6-tribromoisocyanatobenzene) used in Example 5 are shown in the following table.

**[Table 1]**

| | Composition | Y [wt%] |
|---|---|---|
| Synthetic Ex. 2 | 65% of binuclear compound, 23% of a trinuclear compound, 8% of a tetranuclear compound and 4% of pentanuclear and higher nuclear compounds | 54.3 |
| Synthetic Ex. 3 | 100% of a binuclear compound | 39.1 |
| Synthetic Ex. 6 | 100% of a binuclear compound | 56.0 |
| Synthetic Ex. 7 | 45% of binuclear compound, 18% of a trinuclear compound, 17% of a tetranuclear compound and 20% of pentanuclear and higher nuclear compounds | 38.3 |
| Ex. 5 | 100% of a mononuclear compound | 67.1 |

### EXAMPLE 1

1.0 g of the product of Synthetic Example 2, 9.0 g of granular activated carbon Shirasagi KL (Osaka Gas Chemicals Co., Ltd.) and 40 mL of tetrahydrofuran were stirred in a 200 mL recovery flask using a stir bar at room temperature for 30 minutes, and after withdrawal of the stir bar, the solvent was removed by filtration. The recovered solid was dried under vacuum under heat at 100°C for 8 hours to yield 10.0 g of the product of Synthetic Example 2 supported on activated carbon (a composition of the present invention and an active hydrogen-containing organic compound scavenger of the present invention).

### EXAMPLE 2

1.0 g of the product of Synthetic Example 2, 9.0 g of granular activated carbon Shirasagi WH2C8/32 (Osaka Gas Chemicals Co., Ltd.) and 40 mL of tetrahydrofuran were stirred in a 200 mL recovery flask using a stir bar at room temperature for 30 minutes, and after withdrawal of the stir bar, the solvent was removed by filtration. The recovered solid was dried under vacuum under heat at 100°C for 8 hours to yield 10.0 g of the product of Synthetic Example 2 supported on activated carbon (a composition of the present invention and an active hydrogen-containing organic compound scavenger of the present invention).

### EXAMPLE 3

1.0 g of the product of Synthetic Example 2, 9.0 g of granular activated carbon Shirasagi X7100H-3DRY (Osaka Gas Chemicals Co., Ltd.) and 40 mL of tetrahydrofuran were stirred in a 200 mL recovery flask using a stir bar at room temperature for 30 minutes, and after withdrawal of the stir bar, the solvent was removed by filtration. The recovered solid was dried under vacuum under heat at 100°C for 8 hours to yield 10.0 g of the product of Synthetic Example 2 supported on activated carbon (a composition of the present invention and an active hydrogen-containing organic compound scavenger of the present invention) as a black powder.

### EXAMPLE 4

A 20 mL glass vial was loaded with 20 mg of fine powder of the product of Synthetic Example 2 (an active hydrogen-containing organic compound scavenger of the present invention) and filled with a gas containing 10 ppm of 2-ethyl-1-hexanol (in nitrogen). The vial was left to stand at room temperature for 3 hours. Thereafter, the 2-ethyl-1-hexanol concentration in the gas in the vial was determined by GCMS (electronic nose system HERACLES II manufactured by Alpha M.O.S. Japan), and the results indicated that 81% of the 2-ethyl-1-hexanol was captured. After the standing, the atmosphere in the vial was replaced with nitrogen gas, and the vial was heated at 60°C for 30 minutes. Thereafter, 2-ethyl-1-hexanol was determined by GCMS, and the results indicated that 0% of the captured 2-ethyl-1-hexanol was released.

### EXAMPLE 5

A 20 mL glass vial was loaded with 20 mg of 2,4,6-tribromoisocyanatobenzene (having a bromine content of 67.1 wt%, synthesized by isocyanation of 2,4,6-tribromoaniline (FUJIFILM Wako Pure Chemical Corporation)) (an active hydrogen-containing organic compound scavenger of the present invention in the form of fine powder) and filled with a gas containing 10 ppm of 2-ethyl-1-hexanol. The vial was left to stand at room temperature for 3 hours. Thereafter, the 2-ethyl-1-hexanol concentration in the gas in the vial was determined by GCMS (electronic nose system HERACLES II manufactured by Alpha M.O.S. Japan), and the results indicated that 90% of the 2-ethyl-1-hexanol was captured. After the standing, the atmosphere in the vial was replaced with nitrogen gas, and the vial was heated at 60°C for 30 minutes. Thereafter, 2-ethyl-1-hexanol was determined by GCMS, and the results indicated that 0% of the captured 2-ethyl-1-hexanol was released.

### EXAMPLE 6

The procedure in Example 4 was followed except that a gas containing 10 ppm of 2-methyl-1-butanol (in nitrogen) was used instead of the gas containing 10 ppm of 2-ethyl-1-hexanol (in nitrogen) to assess removal and release of 2-methyl-1-butanol. The results indicated that 89% of the 2-methyl-1-butanol was captured and that 0% of the captured 2-methyl-1-butanol was released.

### EXAMPLE 7

The procedure in Example 4 was followed except that a gas containing 10 ppm of 2-methyl-1-proptanol (in nitrogen) was used instead of the gas containing 10 ppm of 2-ethyl-1-hexanol (in nitrogen) to assess removal and release of 2-methyl-1-proptanol. The results indicate that 48% of the 2-methyl-1-propanol was captured and that 0% of the captured 2-methyl-1-propanol was released.

### COMPARATIVE EXAMPLE 1

The procedure in Example 4 was followed except that an empty vial (not loaded with 20 mg of the product of Synthetic Example 2) was filled with a gas containing 10 ppm of 2-ethyl-1-hexanol (in nitrogen) to assess removal of 2-ethyl-1-hexanol. The results indicate that 0% of the 2-ethyl-1 -hexanol was captured.

### COMPARATIVE EXAMPLE 2

The procedure in Example 4 was followed except that 20 mg of β-cyclodextrin (Tokyo Chemical Industry Co., Ltd.) was used instead of 20 mg of the product of Synthetic Example 2 to assess removal and release of 2-ethyl-1-hexanol. The results indicated that 0% of the 2-ethyl-1-hexanol was captured.

### COMPARATIVE EXAMPLE 3

The procedure in Example 4 was followed except that 5 mg of activated carbon (powder of reagent grade manufactured by FUJIFILM Wako Pure Chemical Corporation) was used instead of 20 mg of the product of Synthetic Example 2 to assess removal and release of 2-ethyl-1-hexanol. The results indicated that 99% of the 2-ethyl-1-hexanol was captured, and 13% of the captured 2-ethyl-1-hexanol was released.

### COMPARATIVE EXAMPLE 4

The procedure in Example 4 was followed except that 5 mg of activated carbon (powder of reagent grade manufactured by FUJIFILM Wako Pure Chemical Corporation) was used instead of 20 mg of the product of Synthetic Example 2, and a gas containing 10 ppm of 2-methyl-1-butanol (in nitrogen) was used instead of the gas containing 10 ppm of 2-ethyl-1-hexanol (in nitrogen) to assess removal and release of 2-methyl-1-butanol. The results indicated that 92% of the 2-methyl-1-butanol was captured, and 6% of the captured 2-methyl-1-butanol was released.

### COMPARATIVE EXAMPLE 5

The procedure in Example 4 was followed except that 5 mg of activated carbon (powder of reagent grade manufactured by FUJIFILM Wako Pure Chemical Corporation) was used instead of 20 mg of the product of Synthetic Example 2, and a gas containing 10 ppm of 2-methyl-1-propanol (in nitrogen) was used instead of the gas containing 10 ppm of 2-ethyl-1-hexanol (in nitrogen) to assess removal and release of 2-methyl-1-propanol. The results indicated that 95% of the 2-methyl-1-propanol was captured, and 12% of the captured 2-methyl-1-propanol was released.

### COMPARATIVE EXAMPLE 6

The procedure in Example 4 was followed except that 5 mg of CARiACT Q-50 (manufactured by FUJI SILYSIA CHEMICAL LTD.) was used instead of 20 mg of the product of Synthetic Example 2 to assess removal and release of 2-ethyl-1-hexanol. The results indicated that 86% of the 2-ethyl-1-hexanol was captured, and 25% of the captured 2-ethyl-1-hexanol was released.

### COMPARATIVE EXAMPLE 7

The procedure in Example 4 was followed except that 5 mg of silica gel NIPGEL AY-001 (Tosoh Silica Corporation) was used instead of 20 mg of the product of Synthetic Example 2 to assess removal and release of 2-ethyl-1-hexanol. The results indicated that 92% of the 2-ethyl-1-hexanol was captured, and 21% of the captured 2-ethyl-1-hexanol was released.

**[Table 2]**

| Item | Scavenger | Removal and release of alcohol | |
|---|---|---|---|
| | | Removal (%) during 3 hours at room temperature | Release (%) during 30 min at 60°C |
| Example 4 | Product of Synthetic Example 2 | 81 (2-Ethyl-1-hexanol) | 0 |
| Example 5 | 2,4,6-Tribromoisocyanatobenzene | 90 (2-Ethyl-1-hexanol) | 0 |
| Example 6 | Product of Synthetic Example 2 | 89 (2-Ethyl-1-butanol) | 0 |
| Example 7 | Product of Synthetic Example 2 | 48 (2-Ethyl-1-propanol) | 0 |
| Comparative Example 1 | Nil | 0 (2-Ethyl-1-hexanol) | - |
| Comparative Example 2 | β-Cyclodextrin | 0 (2-Ethyl-1-hexanol) | - |
| Comparative Example 3 | Activated carbn | 99 (2-Ethyl-1-hexanol) | 13 |
| Comparative Example 4 | Activated carbn | 92 (2-Ethyl-1-butanol) | 6 |
| Comparative Example 5 | Activated carbn | 95 (2-Ethyl-1-propanol) | 12 |
| Comparative Example 6 | Silica gel CARiACT Q-50 | 86 (2-Ethyl-1-hexanol) | 25 |
| Comparative Example 7 | Silica gel NIPGEL AY-001 | 92 (2-Ethyl-1-hexanol) | 21 |

### EXAMPLE 8

30 mg of the product of Synthetic Example 2 (an active hydrogen-containing organic compound scavenger of the present invention) was stirred with 10 mL of water using a stir bar in a capped 20 mL vial at room temperature for 3 months, then recovered as a brown powder by filtering off the water and analyzed by FT-IR. The FT-IR spectrum showed no change in peak intensity or peak pattern from that of the initial sample, which indicates that the sample did not hydrolyze during the long-term contact with water. The results are shown in Table 3.

### EXAMPLE 9

100 mg of the product of Synthetic Example 2 (an active hydrogen-containing organic compound scavenger of the present invention) was stirred using a stir bar in an uncapped 20 mL vial at 60°C in the atmosphere for 2 months, and the resulting brown powder was analyzed by FT-IR. The FT-IR spectrum showed no change in peak intensity or peak pattern from that of the initial sample, which indicates that the isocyanate groups did not hydrolyze with water during the long-term contact with the atmosphere containing a small amount of water under heat. The results are shown in Table 3.

### EXAMPLE 10

30 mg of the product of Synthetic Example 2 (an active hydrogen-containing organic compound scavenger of the present invention) was stirred with 10 mL of 30% aqueous ethanol using a stir bar in a capped 20 mL vial at 45°C for 3 days, then recovered as a brown powder by filtering off the solvent and analyzed by FT-IR. The FT-IR spectrum contained a carbonyl peak attributable to the reaction product of ethanol and the isocyanato group, instead of the isocyanate peak contained in the spectrum of the initial sample, and did not contain a peak attributable to a hydrolysis product, which indicates that the isocyanate groups in the product of Synthetic Example 2 reacted selectively with ethanol and did not react with water. The results are shown in Table 3.

### EXAMPLE 11

30 mg of the product of Synthetic Example 2 (an active hydrogen-containing organic compound scavenger of the present invention) was stirred with 5 mL of toluene and 5 mL of 5% aqueous ethanol using a stir bar in a capped 20 mL vial at 45°C for 3 days, then recovered as a brown powder by evaporating toluene to dryness and analyzed by FT-IR. The FT-IR spectrum contained a carbonyl peak attributable to the reaction product of ethanol and the isocyanato group, instead of the isocyanate peak contained in the spectrum of the initial sample, and did not contain a peak attributable to a hydrolysis product, which indicates that the isocyanate groups in the product of Synthetic Example 2 reacted selectively with ethanol and did not react with water. The results are shown in Table 3.

### EXAMPLE 12

30 mg of the product of Synthetic Example 3 (an active hydrogen-containing organic compound scavenger of the present invention) was stirred with 30% aqueous ethanol using a stir bar in a capped 20 mL vial at 45°C for 3 days, then recovered as a powder by filtering off the solvent and analyzed by FT-IR. The FT-IR spectrum contained a carbonyl peak, instead of the isocyanate peak, which indicates capture of ethanol through the reaction of the isocyanate groups and ethanol, and did not contain a peak attributable to a hydrolysis product, which indicates that the isocyanate groups in the product of Synthetic Example 3 reacted selectively with ethanol and did not react with water. The results are shown in Table 3.

### EXAMPLE 27

30 mg of the product of Synthetic Example 6 (an active hydrogen-containing organic compound scavenger of the present invention) was stirred with 10 mL of water using a stir bar in a capped 20 mL vial at room temperature for 3 months, then recovered as a brown powder by filtering off the water and analyzed by FT-IR. The FT-IR spectrum showed no change in peak intensity or peak pattern from that of the initial sample, which indicates that the sample did not hydrolyze during the long-term contact with water. The results are shown in Table 3.

### EXAMPLE 28

100 mg of the product of Synthetic Example 6 (an active hydrogen-containing organic compound scavenger of the present invention) was stirred using a stir bar in an uncapped 20 mL vial at 60°C in the atmosphere for 2 months, and the resulting brown powder was analyzed by FT-IR. The FT-IR spectrum showed no change in peak intensity or peak pattern from that of the initial sample, which indicates that the isocyanate groups did not hydrolyze with water during the long-term contact with the atmosphere containing a small amount of water under heat. The results are shown in Table 3.

### EXAMPLE 29

30 mg of the product of Synthetic Example 6 (an active hydrogen-containing organic compound scavenger of the present invention) was stirred with 10 mL of 30% aqueous ethanol using a stir bar in a capped 20 mL vial at 45°C for 3 days, then recovered as a brown powder by filtering off the solvent and analyzed by FT-IR. The FT-IR spectrum contained a carbonyl peak attributable to the reaction product of ethanol and the isocyanato group, instead of the isocyanate peak contained in the spectrum of the initial sample, and did not contain a peak attributable to a hydrolysis product, which indicates that the isocyanate groups in the product of Synthetic Example 6 reacted selectively with ethanol and did not react with water. The results are shown in Table 3.

### EXAMPLE 30

30 mg of the product of Synthetic Example 6 (an active hydrogen-containing organic compound scavenger of the present invention) was stirred with 5 mL of toluene and 5 mL of 5% aqueous ethanol using a stir bar in a capped 20 mL vial at 45°C for 3 days, then recovered as a brown powder by evaporating toluene to dryness and analyzed by FT-IR. The FT-IR spectrum contained a carbonyl peak attributable to the reaction product of ethanol and the isocyanato group, instead of the isocyanate peak contained in the spectrum of the initial sample, and did not contain a peak attributable to a hydrolysis product, which indicates that the isocyanate groups in the product of Synthetic Example 6 reacted selectively with ethanol and did not react with water. The results are shown in Table 3.

### COMPARATIVE EXAMPLE 8

30 mg of polymeric MDI (Millionate MR-200, manufactured by Tosoh Corporation) was stirred with 10 mL of water using a stir bar in a capped 20 mL vial at room temperature for 1 month, then recovered as a solid by filtering off the water and analyzed by FT-IR. The FT-IR spectrum was quite different in peak intensity and peak pattern from that of the initial sample, which indicates that MDI hydrolyzed during the contact with water. The results are shown in Table 3.

### COMPARATIVE EXAMPLE 9

100 mg of polymeric MDI (Millionate MR-200, manufactured by Tosoh Corporation) was stirred using a stir bar in an uncapped 20 mL vial at 60°C in the atmosphere for 14 days, and the resulting solid was analyzed by FT-IR. The FT-IR spectrum was quite different in peak intensity and peak pattern from that of the initial sample, which indicates that the polymeric MDI hydrolyzed during the contact with the atmosphere containing a small amount of water under heat. The results are shown in Table 3.

### COMPARATIVE EXAMPLE 10

30 mg of polymeric MDI (Millionate MR-200, manufactured by Tosoh Corporation) was stirred with 30% aqueous ethanol using a stir bar in a capped 20 mL vial at 45°C for 3 days, then recovered as a solid by filtering off the solvent and analyzed by FT-IR. The FT-IR spectrum contained a carbonyl peak attributable to the reaction product of ethanol and the polymeric MDI, instead of the isocyanate peak contained in the spectrum of the initial sample, and also contained a peak attributable to a hydrolysis product of the polymeric MDI, which indicates that the polymeric MDI showed no reaction selectivity between water and ethanol. The results are shown in Table 3.

### COMPARATIVE EXAMPLE 11

30 mg of 2,4,6-tri-tertiary-butylisocyanatobenzen was stirred with 30% aqueous ethanol using a stir bar in a capped 20 mL vial at 45°C for 3 days, then recovered as a solid by filtering off the solvent and analyzed by FT-IR. The FT-IR spectrum contained the isocyanate peak, like the spectrum of the initial sample, and had no difference from that of the initial sample in the shape of other peaks, which indicates that 2,4,6-tri-tertiary-butylisocyanatobenzene did not react with water or ethanol. The results are shown in Table 3.

From Table 3, it is clear that scavengers comprising an isocyanate compound represented by the formula (1) are resistant to ambient water and can capture alcohol compounds.

**[Table 3]**

| Item | Scavenger | Reactivity assessment condition | Results of IR analysis | |
|---|---|---|---|---|
| | | | Reaction with water (hydrolysis) | Reaction with alcohol |
| Example 8 | Product of Synthetic Example 2 | In water | No | - |
| Example 9 | Product of Synthetic Example 2 | In atmosphere | No | - |
| Example 10 | Product of Synthetic Example 2 | In aqueous ethanol | No | Occurred |
| Example 11 | Product of Synthetic Example 2 | In aqueous ethanol | No | Occurred |
| Example 12 | Product of Synthetic Example 3 | In aqueous ethanol | No | Occurred |
| Example 27 | Product of Synthetic Example 6 | In water | No | - |
| Example 28 | Product of Synthetic Example 6 | In atmosphere | No | - |
| Example 29 | Product of Synthetic Example 6 | In aqueous ethanol | No | Occurred |
| Example 30 | Product of Synthetic Example 6 | In aqueous ethanol | No | Occurred |
| Comparative Example 8 | Polymeric MDI | In water | Occurred | - |
| Comparative Example 9 | Polymeric MDI | In atmosphere | Occurred | |
| Comparative Example 10 | Polymeric MDI | In aqueous ethanol | Occurred | Occurred |
| Comparative Example 11 | 2,4,6-Tri-tertiary-butylisocyanatobenzene | In aqueous ethanol | No | No |

### EXAMPLE 13 (silica gel-supported scavenger 1 CARiACT Q-10)

1.0 g of the product of Synthetic Example 2, 9.0 g of silica gel CARiACT Q-10 (FUJI SILYSIA CHEMICAL LTD.) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on silica gel (a composition of the present invention and an active hydrogen-containing organic compound scavenger of the present invention).

### EXAMPLE 14 (silica gel-supported scavenger 2 CARiACT G-10)

0.5 g of the product of Synthetic Example 2, 9.5 g of silica gel CARiACT G-10 (FUJI SILYSIA CHEMICAL LTD.) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on silica gel (a composition of the present invention and an active hydrogen-containing organic compound scavenger of the present invention).

### EXAMPLE 15 (silica gel-supported scavenger 3 NIPGEL AY-001)

0.5 g of the product of Synthetic Example 2, 9.5 g of silica gel NIPGEL AY-001 (Tosoh Silica Corporation) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on silica gel (a composition of the present invention and an active hydrogen-containing organic compound scavenger of the present invention).

### EXAMPLE 16 (silica gel-supported scavenger 4 NIPGEL AY-220D)

0.5 g of the product of Synthetic Example 2, 9.5 g of silica gel NIPGEL AY-220D (Tosoh Silica Corporation) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on silica gel (a composition of the present invention and an active hydrogen-containing organic compound scavenger of the present invention).

### EXAMPLE 17 (silica gel-supported scavenger 4 NIPGEL CX-200)

0.5 g of the product of Synthetic Example 2, 9.5 g of silica gel NIPGEL CX-200 (Tosoh Silica Corporation) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on silica gel (a composition of the present invention and an active hydrogen-containing organic compound scavenger of the present invention).

### EXAMPLE 18 (cellulose-supported scavenger Viscopearl A)

0.5 g of the product of Synthetic Example 2, 9.5 g of cellulose Viscopearl A (Rengo Co., Ltd.) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on cellulose (a composition of the present invention and an active hydrogen-containing organic compound scavenger of the present invention).

### EXAMPLE 19 (silica gel-supported scavenger 5 CARiACT Q-50)

0.5 g of the product of Synthetic Example 3, 9.5 g of silica gel CARiACT Q-50 (FUJI SILYSIA CHEMICAL LTD.) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 3 supported on silica gel (a composition of the present invention and an active hydrogen-containing organic compound scavenger of the present invention).

### EXAMPLE 20 (silica gel-supported scavenger 6 NIPGEL AY-001)

0.5 g of the product of Synthetic Example 3, 9.5 g of silica gel NIPGEL AY-001 (Tosoh Silica Corporation) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 3 supported on silica gel (a composition of the present invention and an active hydrogen-containing organic compound scavenger of the present invention).

### EXAMPLE 31 (silica gel-supported scavenger 7 NIPGEL CX-200)

0.5 g of the product of Synthetic Example 6, 9.5 g of silica gel NIPGEL CX-200 (Tosoh Silica Corporation) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 6 supported on silica gel (a composition of the present invention and an active hydrogen-containing organic compound scavenger of the present invention).

### EXAMPLE 32 (hydrotalcite-supported scavenger KYOWAAD 500)

0.5 g of the product of Synthetic Example 2, 9.5 g of KYOWAAD 500 (hydrotalcite manufactured by Kyowa Chemical Industry Co., Ltd.) and 200 mL of dichloromethane were stirred using a stir bar in a 500 mL recovery flask at room temperature for 30 minutes, and after withdrawal of the stir bar, the solvent was slowly removed on an evaporator under vacuum. The solid residue was dried by blowing nitrogen gas at 60°C for 3 hours and then at room temperature for 24 hours to yield the product of Synthetic Example 2 supported on hydrotalcite (a composition of the present invention and an active hydrogen-containing organic compound scavenger of the present invention).

### [Gas removal assessment]

Active hydrogen-containing organic compound scavengers of the present invention were assessed with the active hydrogen-containing organic compound gas removal assessment system illustrated in Fig. 1, which comprises a reservoir tank (made of SUS and having a 20 L capacity), a gas circulation pump, a column to be filled with a scavenger (made of glass and having a 0.1 L capacity), heaters and so on. The reservoir tank was filled with an active hydrogen-containing organic compound gas (such as ethanol, isopropanol or acetic acid) until an atmosphere (circulating gas) containing a prescribed concentration of the active hydrogen-containing organic compound gas (in nitrogen) was made in the system. The column was packed with 5 g of a scavenger of the present invention and then adjusted to 25°C. The circulating gas adjusted to 25°C with a heater was flown through the column by the circulation pump at a rate of 10 L/min for 3 hours, and the concentration of the active hydrogen-containing organic compound in the reservoir tank after the 3-hour gas circulation was determined. Then, the column packed with the scavenger was adjusted to 60°C with a heater, then the circulating gas adjusted to 60°C was flown through the column by the circulation pump at a rate of 10 L/min for 30 minutes, and the concentration of the active hydrogen-containing organic compound in the reservoir tank after 30-minute gas circulation was determined.

### EXAMPLE 21 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 13 (a composition of the present invention and an active hydrogen-containing organic compound scavenger of the present invention), and an assessment was carried out. The ethanol gas concentration in the system after the 3-hour gas circulation at 25°C was 145 ppm, and the ethanol gas concentration in the system after the 30-minute gas circulation at 60°C was 285 ppm. The 215 ppm concentration drop (= 500 ppm - 285 ppm) corresponds to the amount of ethanol captured by the silica gel-supported scavenger of Example 13 and was not released again. The results are shown in Table 4.

### EXAMPLE 22 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 14 (a composition of the present invention and an active hydrogen-containing organic compound scavenger of the present invention), and an assessment was carried out. The ethanol gas concentration in the system after the 3-hour gas circulation at 25°C was 15 ppm, and the ethanol gas concentration in the system after the 30-minute gas circulation at 60°C was 160 ppm. The 340 ppm concentration drop (= 500 ppm - 160 ppm) corresponds to the amount of ethanol captured by the silica gel-supported scavenger of Example 14 and was not released again. The results are shown in Table 4.

### EXAMPLE 23 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 15 (a composition of the present invention and an active hydrogen-containing organic compound scavenger of the present invention), and an assessment was carried out. The ethanol gas concentration in the system after the 3-hour gas circulation at 25°C was less than 5 ppm, and the ethanol gas concentration in the system after the 30-minute gas circulation at 60°C was 55 ppm. The 445 ppm concentration drop (= 500 ppm - 55 ppm) corresponds to the amount of ethanol captured by the silica gel-supported scavenger of Example 15 and was not released again. The results are shown in Table 4.

### EXAMPLE 24 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 16 (a composition of the present invention and an active hydrogen-containing organic compound scavenger of the present invention), and an assessment was carried out. The ethanol gas concentration in the system after the 3-hour gas circulation at 25°C was 10 ppm, and the ethanol gas concentration in the system after the 30-minute gas circulation at 60°C was 70 ppm. The 430 ppm concentration drop (= 500 ppm - 70 ppm) corresponds to the amount of ethanol captured by the silica gel-supported scavenger of Example 16 irreversibly. The results are shown in Table 4.

### EXAMPLE 25 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 17 (a composition of the present invention and an active hydrogen-containing organic compound scavenger of the present invention), and an assessment was carried out. The ethanol gas concentration in the system after the 3-hour gas circulation at 25°C was less than 5 ppm, and the ethanol gas concentration in the system after the 30-minute gas circulation at 60°C was 15 ppm. The 485 ppm concentration drop (= 500 ppm - 15 ppm) corresponds to the amount of ethanol captured by the silica gel-supported scavenger of Example 17 and was not released again. The results are shown in Table 4.

### EXAMPLE 26 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 20 (a composition of the present invention and an active hydrogen-containing organic compound scavenger of the present invention), and an assessment was carried out. The ethanol gas concentration in the system after the 3-hour gas circulation at 25°C was 10 ppm, and the ethanol gas concentration in the system after the 30-minute gas circulation at 60°C was 50 ppm. The 450 ppm concentration drop (= 500 ppm - 50 ppm) corresponds to the amount of ethanol captured by the silica gel-supported scavenger of Example 20 and was not released again. The results are shown in Table 4.

### EXAMPLE 33 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the silica gel-supported scavenger of Example 31 (a composition of the present invention and an active hydrogen-containing organic compound scavenger of the present invention), and an assessment was carried out. The ethanol gas concentration in the system after the 3-hour gas circulation at 25°C was 5 ppm, and the ethanol gas concentration in the system after the 30-minute gas circulation at 60°C was 15 ppm. The 485 ppm concentration drop (= 500 ppm - 15 ppm) corresponds to the amount of ethanol captured by the silica gel-supported scavenger of Example 31 and was not released again. The results are shown in Table 4.

### EXAMPLE 34 (Assessment of Isopropanol Gas Removal)

The procedure in Example 33 was followed except that the gas species (the target active hydrogen-containing organic compound to be removed) was changed from ethanol to isopropanol. The isopropanol gas concentration in the system after the 3-hour gas circulation at 25°C was 10 ppm, and the isopropanol gas concentration in the system after the 30-minute gas circulation at 60°C was 30 ppm. The 470 ppm concentration drop (= 500 ppm - 30 ppm) corresponds to the amount of isopropanol captured by the silica gel-supported scavenger of Example 31 and was not released again. The results are shown in Table 4.

### EXAMPLE 35 (Assessment of Acetic Acid Gas Removal)

The procedure in Example 33 was followed except that the gas species (the target active hydrogen-containing organic compound to be removed) was changed from ethanol to acetic acid, and the initial acetic acid gas concentration was changed to 15.0 ppm. The acetic acid gas concentration in the system after the 3-hour gas circulation at 25°C was 0.2 ppm, and the acetic acid gas concentration in the system after the 30-minute gas circulation at 60°C was 0.4 ppm. The 14.7 ppm concentration drop (= 15.0 ppm - 0.4 ppm) corresponds to the amount of acetic acid captured by the silica gel-supported scavenger of Example 31 and was not released again. The results are shown in Table 4.

### EXAMPLE 36 (Assessment of Ethanol Gas Removal)

An atmosphere containing 500 ppm ethanol gas (in nitrogen) was made in the active hydrogen-containing organic compound gas removal assessment system. Then, the column in the active hydrogen-containing organic compound gas removal assessment system was filled with the hydrotalcite-supported scavenger of Example 32 (a composition of the present invention and an active hydrogen-containing organic compound scavenger of the present invention), and an assessment was carried out. The ethanol gas concentration in the system after the 3-hour gas circulation at 25°C was 55 ppm, and the ethanol gas concentration in the system after the 30-minute gas circulation at 60°C was 80 ppm. The 420 ppm concentration drop (= 500 ppm - 80 ppm) corresponds to the amount of ethanol captured by the hydrotalcite-supported scavenger of Example 32 and was not released again. The results are shown in Table 4.

### COMPARATIVE EXAMPLE 12

An atmosphere containing 500 ppm ethanol gas (in nitrogen) was made in the active hydrogen-containing organic compound gas removal assessment system. Then, an assessment was carried out with nothing in the column in the active hydrogen-containing organic compound gas removal assessment system (i.e., with the column empty). The ethanol gas concentration in the system after the 3-hour gas circulation at 25°C was 500 ppm, and the ethanol gas concentration in the system after the 30-minute gas circulation at 60°C was 500 ppm. There was no drop in the ethanol concentration in the circulating gas. The results are shown in Table 4.

### COMPARATIVE EXAMPLE 13

An atmosphere containing 15.0 ppm acetic acid gas (in nitrogen) was made in the active hydrogen-containing organic compound gas removal assessment system. Then, an assessment was carried out with nothing in the column in the active hydrogen-containing organic compound gas removal assessment system (i.e., with the column empty). The acetic acid gas concentration in the system after the 3-hour gas circulation at 25°C was 15.0 ppm, and the acetic acid gas concentration in the system after the 30-minute gas circulation at 60°C was 15.0 ppm. There was no drop in the acetic acid concentration in the circulating gas. The results are shown in Table 4.

**[Table 4]**

| Item | Scavenger | Target substance | Target gas concentration (ppm) | |
|---|---|---|---|---|
| | | | After 3-hour circulation at 25°C | After 30-minute circulation at 60°C |
| Example 21 | Product of Synthetic Example 2 / silica gel Q-10 | Ethanol | 145 | 285 |
| Example 22 | Product of Synthetic Example 2 / silica gel G-10 | Ethanol | 15 | 160 |
| Example 23 | Product of Synthetic Example 2 / silica gel YA-001 | Ethanol | less than 5 | 55 |
| Example 24 | Product of Synthetic Example 2 / silica gel YA-220D | Ethanol | 10 | 70 |
| Example 25 | Product of Synthetic Example 2 / silica gel CX-200 | Ethanol | less than 5 | 15 |
| Example 26 | Product of Synthetic Example 3 / silica gel YA-001 | Ethanol | 10 | 50 |
| Example 33 | Product of Synthetic Example 6 / silica gel CX-200 | Ethanol | 5 | 15 |
| Example 34 | Product of Synthetic Example 6 / silica gel CX-200 | Isopropanol | 10 | 30 |
| Example 35 | Product of Synthetic Example 6 / silica gel CX-200 | Acetic acid | 0.2 | 0.4 |
| Example 36 | Product of Synthetic Example 6 / hydrotalcite | Ethanol | 55 | 80 |
| Comparative Example 12 | Nil | Ethanol | 500 | 500 |
| Comparative Example 13 | Nil | Acetic acid | 15 | 15 |

The present invention has been described in detail with reference to specific embodiments. However, it is apparent to those skilled in the art that various changes and modifications are possible without departing from the nature and the scope of the present invention.

The entire disclosures of Japanese Patent Application No. 2020-200086 filed on December 2, 2020 and Japanese Patent Application No. 2021-125610 filed on July 30, 2021 including specifications, claims, drawings and summaries are incorporated herein by reference in their entireties.

### REFERENCE SYMBOLS

10. Reservoir tank (made of SUS and having a capacity of 20 L)
11. Hygrothermograph
12. Gas flowmeter
13. Gas circulation pump
14. Heater
15. Thermometer
16. Manometer
17. Column to be filled with a scavenger
18. Heater
19. Valve
20. Direction of the flow of the circulating gas

### INDUSTRIAL APPLICABILITY

The present invention makes it possible to efficiently remove active hydrogen-containing organic compounds which are difficult to remove by conventional techniques such as alcohol compounds. The active hydrogen-containing organic compound scavenger of the present invention has a long-lasting ability to remove active hydrogen-containing organic compounds irreversibly even in the presence of water and can be used in the environmental energy field, the medical and life science field and in other various scenes.

## Claims

1. An active hydrogen-containing organic compound scavenger comprising an isocyanate compound having a bromine content of from 38 to 78 wt% represented by the following formula (1): 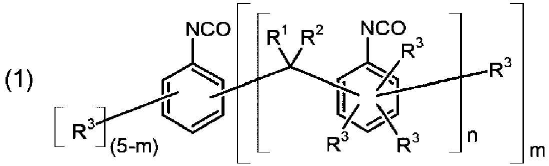 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).

2. The active hydrogen-containing organic compound scavenger according to Claim 1, wherein the isocyanate compound represented by the formula (1) (which may be a single species or a mixture of two or more species, wherein in the case of a mixture, n represents an average n of the mixture) is an isocyanate compound represented by the following formula (1a): 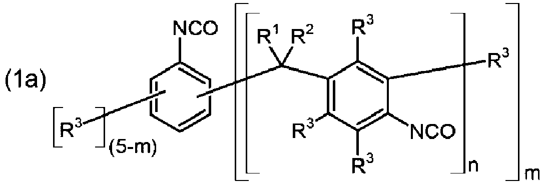 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).

3. The active hydrogen-containing organic compound scavenger according to Claim 1, wherein the isocyanate compound represented by the formula (1) (which may be a single species or a mixture of two or more species, wherein in the case of a mixture, n represents an average n of the mixture) is an isocyanate compound represented by the following formula (1b): 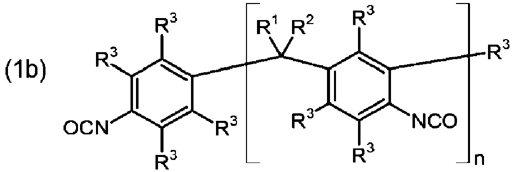 (wherein R¹ and R² each independently represent a hydrogen atom or a C₁-₄ alkyl group, each R³ independently represents a hydrogen atom, a C₁₋₄ alkyl group or a bromine atom, and n represents a real number of 0 or more) (wherein the isocyanate compound may be a single species or a mixture of two or more species, and in the case of a mixture, n represents an average n of the mixture).

4. The active hydrogen-containing organic compound scavenger according to any one of Claims 1 to 3, wherein the bromine content is from 50 to 78 wt%.

5. The active hydrogen-containing organic compound scavenger according to any one of Claims 1 to 4, wherein R¹ and R² each represent a hydrogen atom or a methyl group.

6. The active hydrogen-containing organic compound scavenger according to any one of Claims 1 to 5, wherein R¹ and R² each independently represent a hydrogen atom.

7. The active hydrogen-containing organic compound scavenger according to any one of Claims 1 to 6, wherein each R³ in the formula (1) independently represents a hydrogen atom or a bromine atom.

8. The active hydrogen-containing organic compound scavenger according to any one of Claims 1 to 7, wherein n is a real number of from 0 to 3.

9. A method for removing at least one compound selected from the group consisting of alcohol compounds, thiol compounds, amine compounds, phenol compounds and carboxylic acid compounds, which comprises bringing the at least one compound into contact with the active hydrogen-containing organic compound scavenger as defined in any one of Claims 1 to 8.

10. A composition comprising an isocyanate compound having a bromine content of from 38 to 78 wt% represented by the following formula (1): 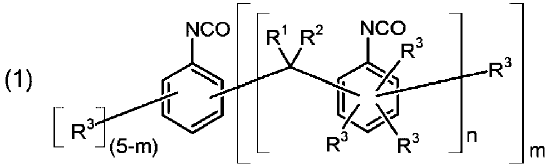 [0025] (wherein R¹ and R² each independently represent a hydrogen atom or a C₁-₄ alkyl group, each R³ independently represents a hydrogen atom, a C₁₋₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture) and a support.

11. The composition according to Claim 10, wherein the isocyanate compound represented by the formula (1) (which may be a single species or a mixture of two or more species, wherein in the case of a mixture, n represents an average n of the mixture) is an isocyanate compound represented by the following formula (1a): 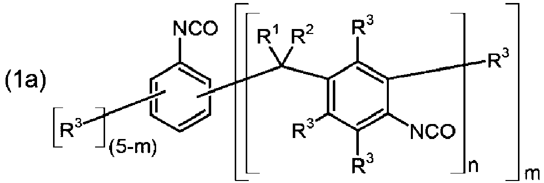 (wherein R¹ and R² each independently represent a hydrogen atom or a C₁-₄ alkyl group, each R³ independently represents a hydrogen atom, a C₁-₄ alkyl group or a bromine atom, m represents at least one selected from the group consisting of 0, 1, 2 and 3, and n represents a real number of 0 or more) (wherein the isocyanate compound may be a single species or a mixture of two or more species, and in the case of a mixture, n represents an average n of the mixture).

12. The composition according to Claim 10, wherein the isocyanate compound represented by the formula (1) (which may be a single species or a mixture of two or more species, wherein in the case of a mixture, n represents an average n of the mixture) is an isocyanate compound represented by the following formula (1b): 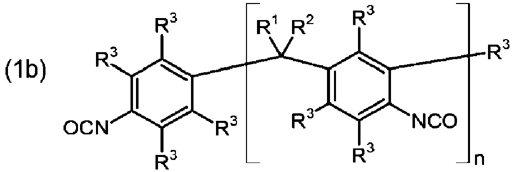 (wherein R¹ and R² each independently represent a hydrogen atom or a C₁-₄ alkyl group, each R³ independently represents a hydrogen atom, a C₁-₄ alkyl group or a bromine atom, and n represents a real number of 0 or more) (wherein the isocyanate compound may be a single species or a mixture of two or more species, and in the case of a mixture, n represents an average n of the mixture).

13. The composition according to any one of Claims 10 to 12, wherein each R³ independently represents a hydrogen atom or a bromine atom.

14. The composition according to any one of Claims 10 to 13, wherein the isocyanate compound is supported on the support.

15. The composition according to any one of Claims 10 to 14, wherein the amount of the isocyanate compound supported on the support is from 0.1 part by weight to 60 parts by weight per 100 parts by weight of the support.

16. The composition according to any one of Claims 10 to 15, wherein the support comprises one or more members of selected from the group consisting of activated carbon, activated clay, diatomaceous earth, a porous resin, non-woven cloth, mesoporous silica, silica gel, aluminosilicate, hydrotalcite, zeolite, activated alumina, titania, magnesia and zirconia.

17. An active hydrogen-containing organic compound scavenger comprising the composition as defined in any one of Claims 10 to 16.

18. A method for removing at least one compound selected from the group consisting of alcohol compounds, thiol compounds, amine compounds, phenol compounds and carboxylic acid compounds, which comprises bringing the at least one compound into contact with the composition as defined in any one of Claims 10 to 16.
